(19) Europäisches Patentamt
European Patent Office
Office européen des brevets

(11) Veröffentlichungsnummer: **0 002 459**
**A2**

(12) # EUROPÄISCHE PATENTANMELDUNG

(21) Anmeldenummer: 78101451.9

(22) Anmeldetag: 24.11.78

(51) Int. Cl.²: C 07 D 209/48
C 07 D 207/40, C 07 D 207/50
A 01 N 9/22

(30) Priorität: 07.12.77 DE 2754492

(43) Veröffentlichungstag der Anmeldung: 27.06.79 Patentblatt 79/13

(84) Benannte Vertragsstaaten: BE CH DE FR GB NL SE

(71) Anmelder: **Bayer Aktiengesellschaft**
**Zentralbereich Patente, Marken und Lizenzen Bayerwerk**
**D-5090 Leverkusen 1(DE)**

(72) Erfinder: **Kühle, Engelbert, Dr.**
**von-Bodelschwingh-Strasse 42**
**D-5060 Bergisch Gladbach 2(DE)**

(72) Erfinder: **Brandes, Wilhelm, Dr.**
**Eichendorffstrasse 3**
**D-5653 Leichlingen 1(DE)**

(72) Erfinder: **Frohberger, Paul-Ernst, Dr.**
**Willi-Baumeister-Strasse 5**
**D-5090 Leverkusen 1(DE)**

(54) **N-Sulfenylierte Carbamoyl-Verbindungen, Verfahren zu ihrer Herstellung und ihre fungizide Verwendung.**

(57) Die Erfindung betrifft neue N-sulfenylierte Carbamoyl-Verbindungen, ein Verfahren zu ihrer Herstellung sowie ihre Verwendung als Fungizide.

Die Verbindungen der allgemeinen Formel

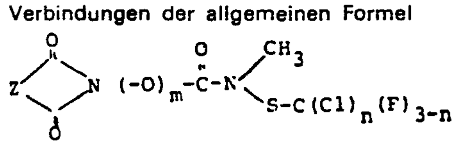

in welcher Z, m und n die in der Beschreibung angegebene Bedeutung besitzen, werden erhalten, wenn man N-sulfenylierte Carbamidsäurefluoride mit einem Dicarbonimid-Derivat in Gegenwart eines Verdünnungsmittels und eines säurebindenden Mittels umsetzt.

Die erfindungsgemäßen Wirkstoffe weisen eine starke fungitoxische Wirkung auf. Eine besonders gute Wirksamkeit entfalten sie gegen parasitäre Pilze auf oberiridischen Pflanzenteilen, wie z.B. gegen Phytophthora-Arten und gegen den Erreger des Apfelschorfs. Sie zeigen ferner eine hohe Wirksamkeit gegen Getreidekrankheiten.

EP 0 002 459 A2

Croydon Printing Company Ltd.

BAYER AKTIENGESELLSCHAFT 5090 Leverkusen,Bayerwerk
Zentralbereich
Patente, Marken und Lizenzen
Slr/Th
I a

N-Sulfenylierte Carbamoyl-Verbindungen, Verfahren zu ihrer Herstellung und ihre fungizide Verwendung

Die vorliegende Erfindung betrifft neue N-sulfenylierte Carbamoyl-Verbindungen, ein Verfahren zu ihrer Herstellung, sowie ihre Verwendung als Fungizide.

Es ist bereits bekannt geworden, daß N-Trihalogenmethansulfenyl-dicarbonimide eine fungizide Aktivität besitzen. So wird z.B. N-Trichlormethansulfenyl-tetrahydrophthalimid seit mehreren Jahren als Blattfungizid in verschiedenen Kulturen praktisch eingesetzt (vgl. US-PS 2 553 770). Bezüglich der Wirksamkeit befriedigt dieses Produkt nicht immer. Weiterhin sind auch N-sulfenylierte Carbamidoxime mit einem Trihalogenmethansulfenyl-Rest fungizid wirksam; auch bei dieser Verbindungsklasse ist die fungidzide Wirkung nicht immer ausreichend (vgl. hierzu DT-OS 22 43 626).

Es wurden als neue Stoffe die N-sulfenylierten Carbamoyl-Verbindungen der allgemeinen Formel

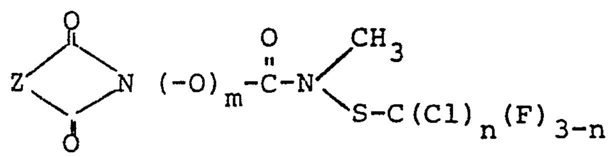

(I)

in welcher

Z für zwei benachbarte C-Atome eines bifunktionellen niederen aliphatischen, eines cycloaliphatischen oder gegebenenfalls substituierten aromatischen Restes steht,

m die Zahlen 0 und 1 und

n die Zahlen 0, 1, 2 und 3 bedeuten,

gefunden. Die Verbindungen weisen starke fungizide Eigenschaften auf.

Weiterhin wurde gefunden, daß man die N-sulfenylierten Carbamoyl-Verbindungen der Formel (I) erhält, wenn man N-sulfenylierte Carbamidsäurefluoride der Formel

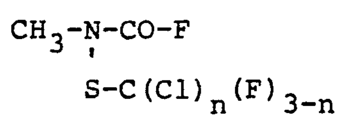

(II)

in welcher n die oben angegebene Bedeutung hat,

mit einem Dicarbonimid-Derivat der Formel

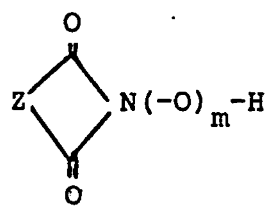

(III)

in welcher Z und m die oben angegebene Bedeutung haben,

in Gegenwart eines Verdünnungsmittels und eines säurebindenden Mittels umsetzt.

Es ist als überraschend zu bezeichnen, daß die erfindungsgemäßen Verbindungen eine höhere fungizide Wirkung aufweisen als die zum Stand der Technik genannten Verbindungen. Die gefundenen neuen Verbindungen stellen somit eine Bereicherung der Technik dar.

Der Reaktionsverlauf läßt sich bei Verwendung von Phthalimid und N-(Fluordichlormethylsulfenyl)-methylcarbamidsäurefluorid als Ausgangsmaterialien durch folgendes Formelchema wiedergeben:

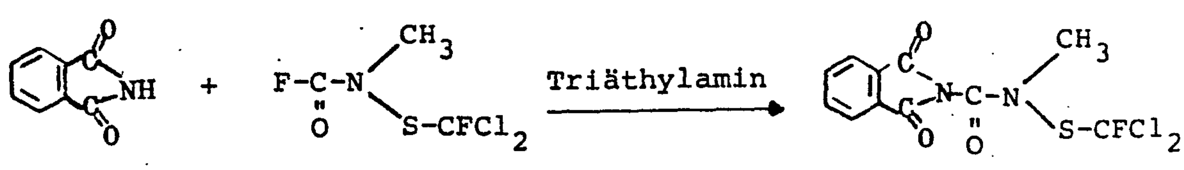

Die als Ausgangsmaterial zu verwendenden N-sulfenylierten Methylcarbamidsäure-fluoride sind durch die Formel (II) definiert. In dieser Formel steht n vorzugseise für die Zahlen 2 und 3. Diese Verbindungen sind bekannt (vgl. DT-AS 1 297 095 /LeA 10857/ bzw. die entsprechende US-PS 3 639 471).

Die zur Umsetzung weiterhin benötigten Dicarbonimid-Derivate sind durch die Formel (III) definiert. In dieser Formel steht Z vorzugsweise für einen niederaliphatischen bifunktionellen Rest mit 2 bis 4 C-Atomen, einen cycloaliphatischen Fünf- oder Sechsring mit 5 bis 9 C-Atomen oder einen aromatischen Rest mit 6 bis 10 C-Atomen, der gegebenenfalls durch Nitro, Chlor, Fluor oder einen Niederalkylrest substituiert sein kann. Als Beispiele für Verbindungen der Formel (III) seien genannt die Imide oder Oximide der Malein-, Bernstein-, Itacon- oder Citraconsäure, ferner der Dihydro-, Tetrahydro-, Hexahydro- und 4-Methyltetrahydro-phthalsäure, der Phthalsäure, 3-Chlorphthalsäure, 4-Nitrophthalsäure, 3-Methylphthalsäure und der Naphthalin-2,3-dicarbonsäure. Die Verbindungen und deren Darstellung sind allgemein bekannt.

Als Verdünnungsmittel kommen alle inerten organischen Lösungsmittel in Frage. Hierzu gehören vorzugsweise Äther, wie Diäthyläther, Tetrahydrofuran und Dioxan; Kohlenwasserstoffe, wie Toluol; Chlorkohlenwasserstoffe, wie Chloroform und Chlorbenzol.

Zur Bindung des bei der Reaktion entstehenden Fluorwasserstoffes setzt man dem Reaktionsgemisch ein Säurebindemittel zu. Bevorzugt verwendet man eine tertiäre Aminbase, wie Triäthylamin, oder aber anorganische Basen, wie Alkalihydroxide oder -carbonate. Man kann auch direkt die Alkalisalze der Dicarbonsäureimide bzw. -oximide in wäßriger Phase umsetzen.

Die Reaktionstemperaturen können in einem größeren Bereich variiert werden, im allgemeinen arbeitet man zwischen o und 100°C, vorzugsweise bei 20 bis 50°C.

Bei der Durchführung des Verfahrens arbeitet man im allgemeinen in molaren Mengen. In manchen Fällen hat es sich als vorteilhaft erwiesen, wenn man das sulfenylierte Methylcarbamidsäure-fluorid in geringem Überschuß bis zu etwa 20% einsetzt.

Die Aufarbeitung des Reaktionsgemisches erfolgt in üblicher Weise. Die Reaktionsprodukte sind meistens kristalline Verbindungen, die nach Zugabe von Wasser abgetrennt und nötigenfalls umgelöst werden können.

Die erfindungsgemäßen Wirkstoffe weisen eine starke fungitoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen und Bakterien geeignet. Fungitoxische Mittel im Pflanzenschutz werden eingesetzt zur Bekämpfung von Plasmodiophoro-

mycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger.

Eine besonders gute Wirksamkeit entfalten sie gegen parasitäre Pilze auf oberirdischen Pflanzenteilen, wie z.B. gegen Phytophthora-Arten und gegen den Erreger des Apfelschorfs (Fusicladium dendriticum). Sie zeigen ferner eine hohe Wirksamkeit gegen Getreidekrankheiten, wie gegen Weizensteinbrand und Getreiderost.

Als Pflanzenschutzmittel können die erfindungsgemäßen Wirkstoffe zur Saatgutbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen,

Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozenten, vorzugsweise zwischen 0,05 und 0,0001 %.

Bei der Saatgutbehandlung werden im allgemeinen Wirkstoffmengen von 0,001 bis 50 g je Kilogramm Saatgut, vorzugsweise 0,01 bis 10 g benötigt.

Die nachfolgenden Beispiele zeigen Verwendungsmöglichkeiten für die erfindungsgemäßen Wirkstoffe auf.

Beispiel A:

Phytophthora-Test (Tomaten) / Protektiv

| | |
|---|---|
| Lösungsmittel | 4,7 Gewichtsteile Aceton |
| Emulgator: | 0,3 Gewichtsteile Alkyl-aryl-polyglykoläther |
| Wasser: | 95 Gewichtsteile |

Man vermischt die für die gewünschte Wirkstoffkonzentration in der Spriztflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70 % im Gewächshaus. Anschließend werden die Tomatenpflanzen mit einer wäßrigen Sporensuspension von Phytophthora infestans inokuliert. Die Pflanzen werden in eine Feuchtkammer mit einer 100 %igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Die Versuchsauswertung ergab, daß insbesondere die folgenden erfindungsgemäßen Verbindungen bekannten Vergleichspräparaten (z.B. dem N-Methyl-N-fluordichlormethylthio-carbamidsäureester des Benzhydroximsäurecyanids) überlegen sind:

Verbindungen gemäß Herstellungsbeispielen 5, 4, 11, 3, 6, 7, 8, 2, 12, 13.

Beispiel B:

Fusicladium-Test (Apfelschorf) / Protektiv

| | |
|---|---|
| Lösungsmittel: | 4,7 Gewichtsteile Aceton |
| Emulgator: | 0,3 Gewichtsteile Alkyl-aryl-polyglykoläther |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4 - 6 Blattstadium befinden, bis zur Tropfnässe. Die Pflanzen verbleiben 24 Stunden bei 20°C und einer relativen Luftfeuchtigkeit von 70 % im Gewächshaus. Anschließend werden sie mit einer wäßrigen Konidiensuspension des Apfelschorferregers (Fusicladium dendriticum Fuck.) inokuliert und 18 Stunden lang in einer Feuchtkammer bei 18 bis 20°C und 100 % relativer Luftfeuchtigkeit inkubiert.

Die Pflanzen kommen dann erneut für 14 Tage in Gewächshaus.

15 Tage nach der Inokulation wird der Befall der Sämlinge in % bestimmt.

Die Versuchsauswertung ergab, daß insbesondere die folgenden erfindungsgemäßen Verbindungen bekannten Vergleichspräparaten (z.B. dem N-Trichlormethansulfenyl-tetrahydrophthalimid) überlegen sind:

Verbindungen gemäß Herstellungsbeispielen 11, 3, 13.

<u>Beispiel C:</u>

Sproßbehandlungs-Test / Getreiderost / protektiv

(blattzerstörende Mykose)

Zur Herstellung einer zweckmäßigen Wirkstoffzubereitung nimmt man C, 25 Gewichtsteile Wirkstoff in 25 Gewichtsteilen Dimethylformamid und 0,06 Gewichtsteilen (Alkylaryl-polyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100 %igen Luftfeuchtigkeit in ein Gewächshaus.

Nach 10 Tagen Verweilzeit der Pflanzen bei einer Temperatur von 20°C und einer Luftfeuchtigkeit von 80 bis 90 % wertet man den Besatz der Pflanzen mit Rostpusteln aus. Der Befallsgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Wirkstoffe, Wirkstoffkonzentrationen in der Spritzbrühe und Befallsgrade werden ermittelt.

Die Versuchsauswertung ergab, daß insbesondere die folgenden erfindungsgemäßen Verbindungen bekannten Vergleichspräparaten (z.B. dem Zinksalz der Äthylen-bis-dithiocarbamidsäure) überlegen sind:

Verbindungen gemäß Herstellungsbeispielen 11, 5, 7.

Beispiel D:

Saatgutbeizmittel-Test / Weizensteinbrand
(samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5 g Chlamydosporen von Tilletia caries pro kg Saatgut. Zur Beizung schüttelt man das Saatgut mit dem Beizmittel in einer verschlossenen Glasflasche. Das Saatgut wird auf feuchtem Lehm unter einer Deckschicht aus einer Lage Mull und 2 cm mäßig feuchter Komposterde 10 Tage lang im Kühlschrank bei 10°C optimalen Keimungsbedingungen für die Sporen ausgesetzt.

Anschließend bestimmt man mikroskopisch die Keimung der Sporen auf den Weizenkörnern, die jeweils mit rund 100 000 Sporen besetzt sind. Der Wirkstoff ist umso wirksamer je weniger Sporen gekeimt sind.

Wirkstoffe, Wirkstoffkonzentrationen mit Beizmittel, Beizmittelaufwandmengen und Keimprozente der Sporen werden ermittelt.

Die Versuchsauswertung ergab, daß insbesondere die folgenden erfindungsgemäßen Verbindungen bekannten Vergleichspräparaten (z.B. dem Zinksalz der Äthylen-bis-dithiocarbamidsäure und dem N-Methyl-N-fluordichlormethylthiocarbamidsäureester des Benzhydroximsäurecyanids) überlegen sind:

Verbindungen gemäß Herstellungsbeispielen 5, 3.

## Herstellungsbeispiele

### Beispiel 1:

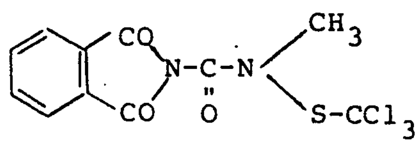

In die Lösung von 14,7 g (0,1 Mol) Phthalimid und 22,5 g (0,1 Mol) N-(Trichlormethylthio)-N-methylcarbamidsäurefluorid in 100 ml Dioxan tropft man bei Raumtemperatur 11 g (0,11 Mol) Triäthylamin ein; hierbei ist kein Temperaturanstieg zu beobachten. Man erhitzt die Lösung während 1 Stunde auf 90°C, kühlt dann ab, destilliert das Lösungsmittel im Vakuum ab und setzt Eiswasser zu. Hierbei kristallisiert der Rückstand aus. Nach dem Umkristallisieren aus Acetonitril erhält man 25 g N-(N-Methyl-N-trichlormethylthio-carbamoyl)-phthalsäureimid vom Fp. 155 bis 157°C.

### Beispiel 2:

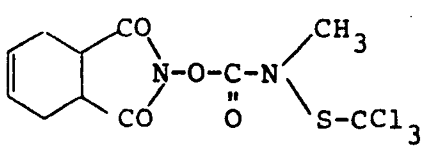

8,3 g (0,05 Mol) $\Delta^4$-Tetrahydrophthal-oximid und 11,8 g (0,05 Mol) N-(Trichlormethylthio)-N-methylcarbamidsäure-

fluorid werden bei 50°C in 80 ml Dioxan gelöst. Beim Zutropften von 6 g Triäthylamin steigt die Temperatur auf 55°C an. Man rührt noch während 1 Stunde bei 60°C und setzt dem Reaktionsgut nach dem Abkühlen Wasser zu, wobei das obige Produkt sich kristallin abscheidet. Nach dem Trocknen erhält man 16,5 g vom Fp. 108 bis 110°C.

In entsprechender Weise wie in den obigen Beispielen beschrieben, können die nachfolgenden Verbindungen der allgemeinen Formel

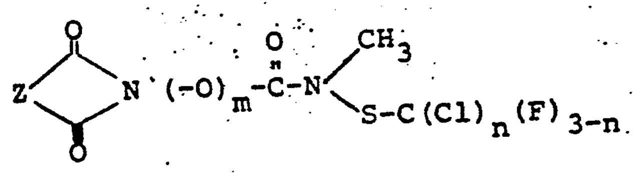 (I)

erhalten werden:

| Beispiel Nr. | Z | m | n | Fp (°C) |
|---|---|---|---|---|
| 3 | 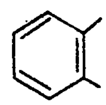 | 0 | 2 | 100-101 |
| 4 | 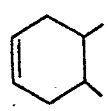 | 0 | 3 | 162-164 |
| 5 | 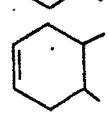 | 0 | 2 | 129-133 |

| Beispiel Nr. | Z | m | n | Fp ($^{o}C$) |
|---|---|---|---|---|
| 6 | 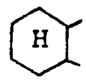 | O | 3 | 125-127 |
| 7 | 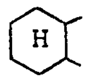 | O | 2 | 105-106 |
| 8 | $CH_2$-$CH_2$ | o | 3 | 116-117 |
| 9 | $CH_2$-$CH_2$ | O | 2 | 111-113 |
| 10 | 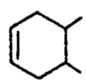 | 1 | 2 | 92-93 |
| 11 | 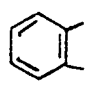 | 1 | 2 | 102-104 |
| 12 | 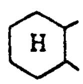 | 1 | 3 | 117-119 |
| 13 | 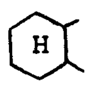 | 1 | 2 | 96-98 |

Patentansprüche

1.) N-Sulfenylierte Carbamoyl-Verbindungen der allgemeinen Formel

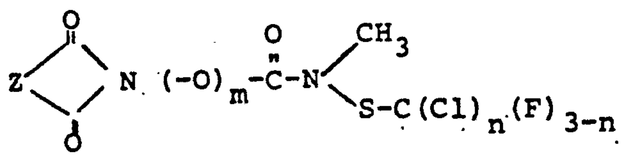

(I)

in welcher

Z für zwei benachbarte C-Atome eines bifunktionellen niederen aliphatischen, eines cycloaliphatischen oder gegebenenfalls substituierten aromatischen Restes steht,

m die Zahlen 0 und 1 und

n die Zahlen 0, 1, 2 und 3 bedeuten.

2.) Verfahren zur Herstellung von N-sulfenylierten Carbamoyl-Verbindungen, dadurch gekennzeichnet, daß man N-sulfenylierte Carbamidsäurefluoride der Formel

$$CH_3-\underset{S-C(Cl)_n(F)_{3-n}}{N}-CO-F \qquad (II)$$

in welcher n die oben angegebene Bedeutung hat,

mit einem Dicarbonimid-Derivat der Formel

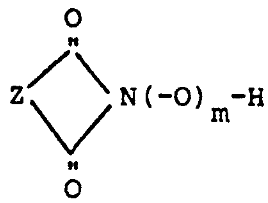

(III)

in welcher Z und m die in Anspruch 1 angegebene Bedeutung haben,

in Gegenwart eines Verdünnungsmittels und eines säurebindenden Mittels umsetzt.

3.) Fungizide Mittel, gekennzeichnet durch einen Gehalt an mindestens einer N-sulfenylierte Carbamoyl-Verbindung gemäß Anspruch 1.

4.) Verfahren zur Bekämpfung von Pilzen, dadurch gekennzeichnet, daß man N-sulfenylierte Carbamoyl-Verbindungen gemäß Anspruch 1 auf Pilze oder ihren Lebensraum einwirken läßt.

5.) Verwendung von N-sulfenylierten Carbamoyl-Verbindungen gemäß Anspruch 1 zur Bekämpfung von Pilzen.

6.) Verfahren zur Herstellung von fungiziden Mitteln, dadurch gekennzeichnet, daß man N-sulfenylierte Carbamoyl-Verbindungen gemäß Anspruch 1 mit Streckmitteln und/oder oberflächenaktiven Mitteln vermischt.